# EUROPEAN PATENT SPECIFICATION

(11) **EP 4 504 024 B1**
(45) Date of publication and mention of the grant of the patent: **11.03.2026**
(21) Application number: 23718811.5
(22) Date of filing: 05.04.2023
(51) Int. Cl.: A61B 1/00

(54) **SYSTEM AND METHOD FOR CLEANING INTERNAL CHANNELS OF AN ENDOSCOPE**
SYSTEM UND VERFAHREN ZUR REINIGUNG DER INNENKANÄLE EINES ENDOSKOPS
SYSTÈME ET PROCÉDÉ DE NETTOYAGE DE CANAUX INTERNES D'ENDOSCOPE

(30) Priority: 06.04.2022 GB 202205049
(43) Date of publication of application: 12.02.2025
(73) Proprietor: Meditech Endoscopy Limited, Tapton, Chesterfield S41 0TZ (GB)
(72) Inventor: RAMSEY, Peter, Tapton, Chesterfield S41 0TZ (GB)
(74) Representative: Dummett Copp LLP
(86) International application number: PCT/GB2023/050914
(87) International publication number: WO 2023/194732

(56) References cited:
- WO-A1-2022/189783
- US-A- 4 576 650
- US-A- 5 749 829
- US-A1- 2020 375 434

## Description

### FIELD OF THE INVENTION

This invention relates to a kit, a system and a method for cleaning internal channels of a medical instrument such as an endoscope. The kit, system and method have particular application during a pre-clean procedure for a medical instrument such as an endoscope. The kit, system and method may be used to flush an air/water channel and/or a biopsy/suction channel of an endoscope.

### BACKGROUND TO THE INVENTION

Endoscopes are now widely used in a number of medical procedures. An endoscope typically includes a light source connector, a control section, an umbilical cord connecting the light source connector to the control section, and an insertion tube terminating at a distal tip. The light source connector provides connections to a light source, an air pump, and a water bottle.

During use of the endoscope, an operator is able to use an air/water valve in the control section to control the delivery of air (from the air pump) or water (from the water bottle) to the distal tip through an air/water channel of the endoscope. A small metal nozzle is generally disposed at the distal tip of the endoscope to deflect air or water from the air/water channel across an image lens of the endoscope. The air/water channel has a small diameter and is actually formed by two separate small diameter channels that only combine into a single channel proximate the distal tip of the endoscope.

The endoscope also includes a suction/biopsy channel. During use of the endoscope a suction valve may be closed (depressed) to allow fluids in the region of the distal tip of the endoscope to be suctioned or drawn up through the suction channel. A biopsy port is also connected to the suction/biopsy channel and allows endotherapy devices, such as devices for performing biopsies, to be inserted into the suction/biopsy channel. The endotherapy devices extend along the suction/biopsy channel in the insertion tube to protrude from the distal tip.

After use of the endoscope it is essential that the endoscope is properly cleaned and sterilised before it is used again. Cleaning refers to the removal of visible debris, which may include both inorganic and organic substances. Cleaning usually involves a mechanical process, together with water and detergent. Following cleaning, sterilisation of the endoscope destroys microorganisms present on or in the endoscope. Sterilisation processes usually involve steam or a chemical sterilant. Following sterilisation, the endoscope undergoes high-level disinfection.

It is currently recommended that a preliminary cleaning routine is undertaken immediately after use of the endoscope and before the light source connector is detached from the light source/video processor. Current pre-clean practice as disclosed e.g. in document US 2020/375434 A1 is to flush air and water through the air/water channel and through the suction/biopsy channel. It is also recommended that the air and water channels and the suction/biopsy channel are irrigated with detergent, not only to expel any blood, mucus and other debris, but also to check for blockages.

It has been found, however, that routine pre-cleaning procedures for endoscopes do not remove biofilm reliably from endoscope channels. This leads to failures in the subsequent decontamination processes.

It is an aim of the present invention to provide a system and method for cleaning internal channels of an endoscope to improve the efficacy of cleaning, sterilisation and disinfection processes.

### SUMMARY OF THE INVENTION

The present invention provides a kit for flushing internal channels of an endoscope, the kit comprising:
a sealing plug for engagement with an air/water cylinder of an endoscope, and the sealing plug being configured, in use, to form a seal at an air inlet of the air/water cylinder and to permit fluid flow between a water inlet of the air/water cylinder and both an air outlet and a water outlet of the air/water cylinder simultaneously;
a detergent delivery assembly for connection to a water bottle connector of the endoscope, the detergent delivery assembly comprising a bag holding a volume of detergent solution, a first fluid conduit extending between a first end disposed within the bag and a second end external to the bag for connection to an air port of the water bottle connector, and a second fluid conduit extending between a first end disposed within the bag and a second end external to the bag for connection to a water port of the water bottle connector; and
a cleaning apparatus comprising a bag holding a volume of detergent solution, the bag having a neck portion for insertion of a distal tip of the endoscope into the bag.

In preferred embodiments the sealing plug is a unitary element having no moving parts.

In some embodiments the kit further comprises a sealing cap for sealing a suction port of the endoscope. The sealing plug and the sealing cap may be parts of a unitary cleaning adaptor made of a resilient polymeric material.

In preferred embodiments the sealing plug comprises:
a tubular stem extending between first and second ends and including a longitudinal passage, the longitudinal passage being open at the first end of said stem;
a head portion closing the longitudinal passage at the second end of said stem and the head portion including a flange extending radially outwardly from the tubular stem;
at least three annular seals extending radially outwardly from the tubular stem and being spaced apart along a length of the tubular stem; and
a transverse passage extending through the tubular stem and providing a fluid flow path between the longitudinal passage and an aperture in an outer surface of the tubular stem, the aperture being disposed between two of the at least three annular seals.

The detergent delivery system may comprise a rupturable or breakable reservoir within an internal volume of the bag, the reservoir holding a volume of a detergent concentrate and the internal volume of the bag holding a volume of water. In some embodiments the reservoir may be provided by a part of the first fluid conduit and a break-to-open feature may be disposed at the first end of the first fluid conduit to seal the reservoir.

In some embodiments the bag of the cleaning apparatus comprises a main body portion providing an internal volume for holding a volume of water, and the cleaning apparatus further comprises a reservoir holding a volume of detergent concentrate, the reservoir being engaged with the neck portion of the bag such that, in use, when the neck portion is opened to access the internal volume of the main body portion, the detergent concentrate is concurrently released from the reservoir to mix with the water.

The present invention further provides a system for flushing internal channels of an endoscope comprising:
a sealing plug engaged with an air/water cylinder of the endoscope, the sealing plug forming a seal at an air inlet of the air/water cylinder while permitting fluid flow between a water inlet of the air/water cylinder and both an air outlet and a water outlet of the air/water cylinder simultaneously;
a detergent delivery assembly connected to a water bottle connector of the endoscope, the detergent delivery assembly comprising a bag holding a volume of detergent solution, a first fluid conduit extending between a first end disposed within the bag and a second end in fluid connection with an air port of the water bottle connector, and a second fluid conduit extending between a first end disposed within the bag and a second end in fluid connection with a water port of the water bottle connector; and
a cleaning apparatus comprising a bag holding a volume of detergent solution, a distal tip of the endoscope being submerged in the volume of detergent solution in the bag.

The system preferably further comprises a sealing cap engaged with and sealing a suction port of the endoscope. The sealing plug and the sealing cap may be parts of a unitary cleaning adaptor made of a resilient polymeric material.

In preferred embodiments a retainer is secured around a part of the endoscope to secure the cleaning apparatus to the endoscope such that the distal tip remains submerged during use. The retainer may comprise a tie, clip, band or tape.

The present invention also provides a method of flushing internal channels of an endoscope using a kit according to the first aspect of the invention, the method comprising:
keeping a light source connector of the endoscope connected to an air pump with the air pump initially switched off;
engaging the sealing plug with an air/water cylinder of the endoscope so that a seal is formed at an air inlet of the air/water cylinder, while permitting fluid flow from a water inlet of the air/water cylinder to both an air outlet and a water outlet of the air/water cylinder simultaneously;
connecting the detergent delivery assembly to a water bottle connector of the endoscope;
inserting a distal tip of the endoscope into the bag of the cleaning apparatus so that the distal tip is submerged in the volume of detergent solution; and
switching on the air pump so that detergent solution from the detergent delivery assembly is forced through an air channel and a water channel of the endoscope.

In preferred embodiments the air pump is switched on for between 30 and 60 seconds.

The method preferably further comprises sealing a suction port of the endoscope with a sealing cap, and switching on a suction pump connected to the light source connector so that liquid from the cleaning apparatus is drawn through a suction channel of the endoscope. The suction pump may be switched on for between 30 and 60 seconds.

In preferred embodiments the method further comprises, after connecting the detergent delivery assembly to the water bottle connector and before switching on the air pump, rupturing or breaking a reservoir within an internal volume of the bag of the detergent delivery assembly so that detergent concentrate held within the reservoir mixes with water in the internal volume of the bag.

In some embodiments the bag of the cleaning apparatus comprises a main body portion providing an internal volume for holding a volume of water, and the cleaning apparatus further comprises a reservoir holding a volume of detergent concentrate, the reservoir being engaged with the neck portion of the bag such that, in use, when the neck portion is opened to access the internal volume of the main body portion, the detergent concentrate is concurrently released from the reservoir to mix with the water. In these embodiments, the method preferably comprises, before inserting the distal tip of the endoscope, opening the neck portion of the bag of the cleaning apparatus to access the internal volume of the main body portion and simultaneously releasing detergent concentrate from the reservoir to mix with the water in the main body portion.

In preferred embodiments the method further comprises switching off the air pump, disconnecting the light source connector from the air pump, and covering the endoscope for transportation. The sealing plug, detergent delivery assembly and cleaning apparatus may remain connected to the endoscope when it is covered.

In some embodiments the method comprises engaging a retainer around a part of the endoscope to secure the cleaning apparatus to the endoscope such that the distal tip remains submerged during use.

Preferred and/or optional features of each aspect and embodiment described above may also be used, alone or in appropriate combination, in the other aspects and embodiments also.

### BRIEF DESCRIPTION OF THE DRAWINGS

The invention will now be further described by way of example only and with reference to the accompanying drawings, in which like reference signs are used for like features, and in which:
Figure 1 illustrates the internal fluid channels of a known endoscope;
Figure 2 illustrates an air/water port and a suction port of a control section of a known endoscope;
Figure 3 illustrates a system for cleaning internal fluid channels of an endoscope according to an embodiment of the present invention;
Figure 4 is a perspective view of an embodiment of a cleaning adaptor of the system of Figure 3;
Figure 5 shows an embodiment of a detergent delivery assembly of the system of Figure 3;
Figure 6 shows an example connector forming part of the detergent delivery assembly of Figure 5 for attaching the cleaning apparatus to the endoscope; and
Figures 7a to 7d illustrate steps in a method of using a cleaning apparatus of the system of Figure 3.

### DESCRIPTION OF THE PREFERRED EMBODIMENTS

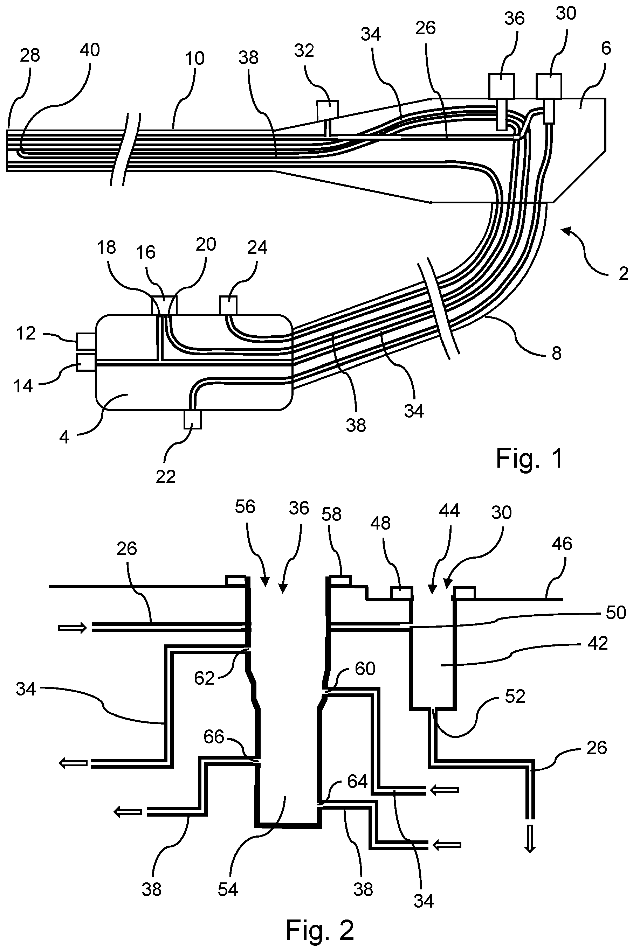

Figure 1 illustrates a known air/water system of an endoscope 2. The endoscope 2 includes a light source connector 4, a control section 6, an umbilical cord 8 connecting the light source connector 4 to the control section 6, and an insertion tube 10. The light source connector 4 comprises a connector 12 for connection to a light source and a connector 14 for connection to an air pump.

The light source connector 4 also includes a connector 16 for attachment of a water bottle. This water bottle connector 16 includes an air port 18 and a water port 20. The light source connector 4 further includes a connection 22 to a suction pump and a connection 24 to a water jet. A suction pump is typically connected to the connection 22 by a tube, which may be referred to as a patient connecting tube. The patient connecting tube extends between the light source connector and a suction jar of the suction pump.

The endoscope 2 includes a suction/biopsy channel 26 that extends from the suction pump connection 22 to a distal end or tip 28 of the insertion tube 10. A first section of the suction/biopsy channel 26 extends from the light source connector 4 to a suction valve housed within a suction port 30 of the control section 6. A second section of the suction/biopsy channel 26 extends from the suction port 30 to the tip 28 of the insertion tube 10. A branch off the second section of the suction/biopsy channel 26 terminates at a biopsy valve in a biopsy port 32. In use fluid and other matter that is drawn along the first section of the suction/biopsy channel 26 passes through the patient connecting tube and into the suction jar of the suction pump.

An air channel 34 of the endoscope extends from the air pump connector 14 to the distal tip 28 of the insertion tube 10. A first section of the air channel 34 extends from the light source connector 4 to an air/water valve housed within an air/water port 36 of the control section 6. A second section of the air channel 34 extends from the air/water port 36 to the tip 28 of the insertion tube 10.

Within the light source connector 4, a branch extends from the first section of the air channel 34 to the air port 18 of the water bottle connector 16 to permit a flow of air from the air channel 34 to a water bottle connected to the connector 16.

A water channel 38 of the endoscope extends from the water port 20 of the water bottle connector 16 to the distal tip 28 of the insertion tube 10. A first section of the water channel 38 extends from the light source connector 4 to the air/water valve housed within the air/water port 36 of the control section 6. A second section of the water channel 38 extends from the air/water port 36 to the tip 28 of the insertion tube 10.

Proximate the distal tip 28 of the insertion tube 10 the air and water channels 34, 38 combine into a single channel. Typically the air and water channels 34, 38 are referred to collectively as the air/water channel 40 of the endoscope 2.

Referring now to Figure 2, the suction port 30 comprises a cylindrical recess or cavity 42 (referred to as a suction cylinder) for receiving the suction valve. The suction cylinder 42 creates an opening 44 in a surface 46 of the control section 6 that is, in this example, surrounded by a raised collar 48. The second section of the suction/biopsy channel 26 enters the suction cylinder 42 at a suction inlet 50 and the first section of the suction/biopsy channel 26 extends from a suction outlet 52 of the suction cylinder 42.

The air/water port 36 comprises a cylindrical recess or cavity 54 (referred to as an air/water cylinder) for receiving the air/water valve. The air/water cylinder 54 creates an opening 56 in the surface 46 of the control section 6 that is surrounded, in this example, by a raised collar 58. The openings 44, 56 of the suction port 30 and air/water port 36 are disposed adjacent each other in the control section 6. The first section of the air channel 34 enters the air/water cylinder 54 at an air inlet 60 and the second section of the air channel 34 extends from an air outlet 62 of the air/water cylinder 54. The first section of the water channel 38 enters the air/water cylinder 54 at a water inlet 64 and the second section of the water channel 38 extends from a water outlet 66 of the air/water cylinder 54.

During use of the endoscope 2 in a medical procedure an air/water valve is seated in the air/water cylinder 54 and a suction valve is seated in the suction cylinder 42. The air/water valve may be covered by a thumb or finger of an operator of the endoscope 2 to direct a flow of air along the air/water channel 40 to the distal tip 28. The air/water valve may be depressed to direct a flow of water along the air/water channel 40 to the distal tip 28. The suction valve may be covered by a thumb or finger of an operator of the endoscope 2 to provide a suction force to draw fluid up through the suction channel 26 away from the tip 28 of the endoscope 2.

Following use of the endoscope 2, the air/water valve and the suction valve are removed from the air/water cylinder 54 and the suction cylinder 42. These valves may be disposable or they may be reusable.

A system according to the present invention is then used to flush the air/water channel 40 and the suction/biopsy channel 26 with a detergent solution.

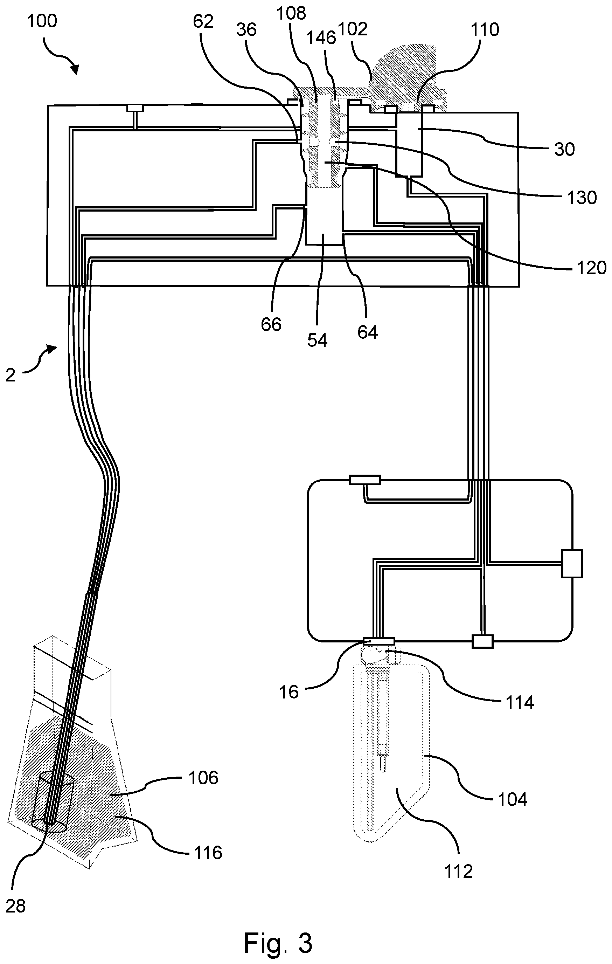

An embodiment of a system 100 according to the present invention is illustrated in Figure 3. The system 100 comprises a cleaning adaptor 102 for insertion into the air/water port 36 of the endoscope 2, a detergent delivery assembly 104 for connection to the water bottle connector 16 of the endoscope 2, and a cleaning apparatus 106 for receiving the distal tip 28 of the endoscope 2.

In one embodiment the cleaning adaptor 102 includes a sealing plug 108 configured to seal the air/water port 36 while permitting a flow of liquid from the water inlet 64 of the air/water cylinder 54 to both the air outlet 62 and the water outlet 66. The cleaning adaptor 102 also includes a cap portion 110 for sealing the suction port 30.

In one embodiment the detergent delivery assembly 104 comprises a bag or pouch 112 for holding a volume of detergent solution, and a connector 114 for connecting the detergent delivery assembly 104 to the water bottle connector 16 of the endoscope 2.

In one embodiment the cleaning apparatus 106 comprises a bag 116 for holding a volume of detergent solution. The cleaning apparatus 106 preferably also includes a retainer for holding or retaining the tip 28 of the endoscope 2 within the volume of detergent solution in the bag during use.

### Cleaning Adaptor

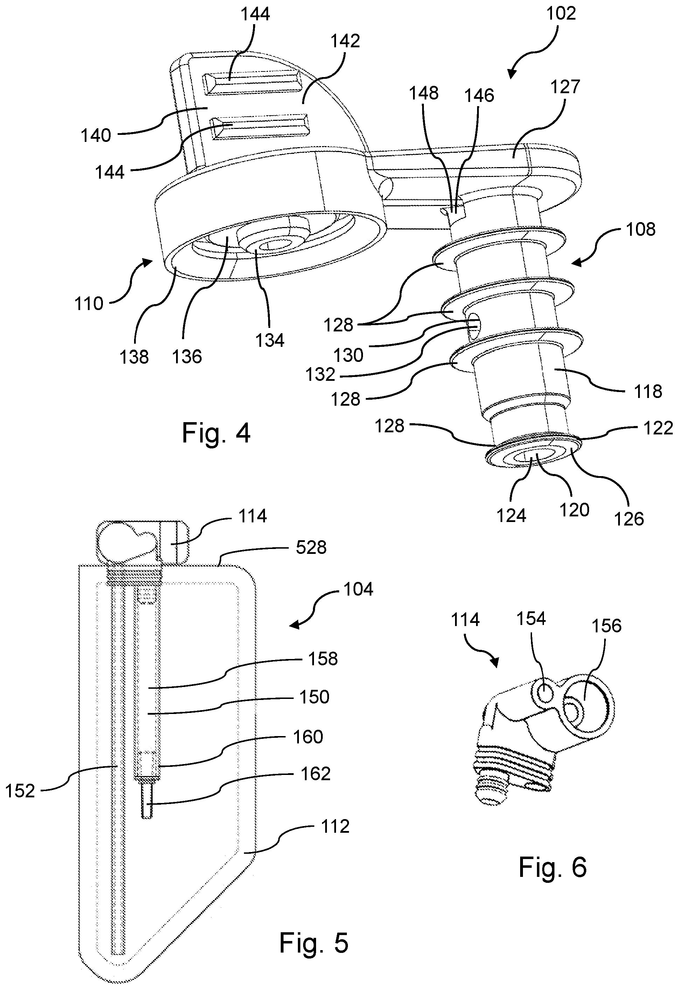

A cleaning adaptor 102 according to a preferred embodiment of the present invention is illustrated in Figure 4. The cleaning adaptor 102 is a unitary, one-piece element and has no moving parts other than due to the inherent flexibility of some parts of the adaptor 102. The cleaning adaptor is preferably made from a suitable polymeric material, having a degree of elasticity or flexibility. The cleaning adaptor may be made from silicone.

The sealing plug 108 of the cleaning adaptor 102 is configured to be inserted into the air/water cylinder 54. The sealing plug 108 comprises an elongate tubular stem 118 having a longitudinal passage 120 terminating at a first end 122 of the stem 118 at an opening 124 in an end surface 126 of the stem 118. A second end of the longitudinal passage 120 is closed by a cover portion or head portion 127.

A plurality of sealing rings or annular seals 128 extend radially outwardly from the stem 118. Each of the annular seals 128 extends circumferentially around the stem 118, and the annular seals 128 are spaced apart along a length of the stem 118. In use the annular seals 128 contact a wall of the air/water cylinder 54 to form a seal. Furthermore, the grip or resistance provided by the annular seals 128 on the wall of the air/water cylinder 54 retains the sealing plug 108 within the air/water cylinder 54 during use.

The sealing plug 108 further comprises a transverse passage 130 terminating in a pair of apertures 132 in the stem 118. The transverse passage 130 intersects the longitudinal passage 120 to permit fluid flow between the longitudinal passage 120 and the transverse passage 130.

In this embodiment the cleaning adaptor 102 also comprises a cap portion 110 for sealing the suction port 30 of the endoscope 2. The cap portion 110 comprises a plug portion 134 and a sealing surface 136 for engagement with the suction cylinder 42. In this embodiment the cap portion 110 further comprises an annular retainer 138 that is configured to engage with the collar 48 surrounding the opening 44 of the suction cylinder 42.

A grip element or grip portion 140 projects from the cap portion 110. The grip element 110 has two opposing grip surfaces 142, and grip features 144 in the form of elongate protrusions or ridges are provided on each of the grip surfaces 142 to assist a user in gripping the grip element 140.

The cleaning adapter 102 also includes a single use feature that prevents subsequent re-use of the adapter 102. In this embodiment, the single use feature comprises a weakened portion 146 proximate a second end of the stem 118 adjacent the head portion 127. A notch or indent 148 is provided in an external surface of the tubular stem 118 at the second end so that a part of a wall of the tubular stem 118 at the second end has a smaller thickness than the rest of the tubular stem 118. This notch 148 or region of reduced thickness does not extend around the full circumference of the stem 118 and is disposed on a side of the tubular stem 118 closest to the cap portion 110. The weakened portion 146 of the stem 118 is designed to rupture when a force is applied to the cleaning adaptor 102 to remove the adaptor 102 from the air/water cylinder 54 after use.

### Detergent Delivery Assembly

A detergent delivery assembly 104 according to a preferred embodiment is shown in Figure 5. The detergent delivery assembly 104 includes a pouch 112 holding a volume of distilled or sterile water. An air inlet conduit 150 extends from a first end within the pouch 112 to a second end engaged with a connector 114 for connecting the detergent delivery assembly 104 to the water bottle connector 16 of the endoscope 2. A liquid outlet conduit 152 extends from a first end within the pouch 112 to a second end engaged with the connector 114.

The connector 114 includes an air port 154 for attaching to the air port 18 of the water bottle connector 16 and a liquid port 156 for attaching to a water port 20 of the water bottle connector 16. The air inlet conduit 150 is preferably fluidly connected to the air port 154 of the connector 114 and the liquid outlet conduit 152 is preferably fluidly connected to the liquid port 156 of the connector 114.

A detergent concentrate reservoir 158 for holding a volume of detergent concentrate is disposed at the first end of the air inlet conduit 150. In this example the detergent concentrate reservoir 158 is continuous with the rest of the air inlet conduit 150 such that the air inlet conduit 150 is in the form of a tube having a constant internal diameter. The internal diameter of the air inlet conduit 150 is preferably greater than an internal diameter of the liquid outlet conduit 152. An end of the detergent concentrate reservoir 158 includes a break-to-open feature 160. In this example the break-to-open feature 160 comprises a break-off moulding including a flat blade tab 162 disposed at the first end of the air inlet conduit 150.

In use, a user grasps a part of the flat blade tab 162 through the pouch 112 and breaks it off from the end of the air inlet conduit 150, thereby releasing detergent concentrate held within the reservoir 158 into the sterile water within the pouch 112.

The detergent concentrate reservoir 158 may contain about 1 ml of detergent concentrate. The pouch 112 may contain about 30 ml of sterile water.

### Cleaning Apparatus

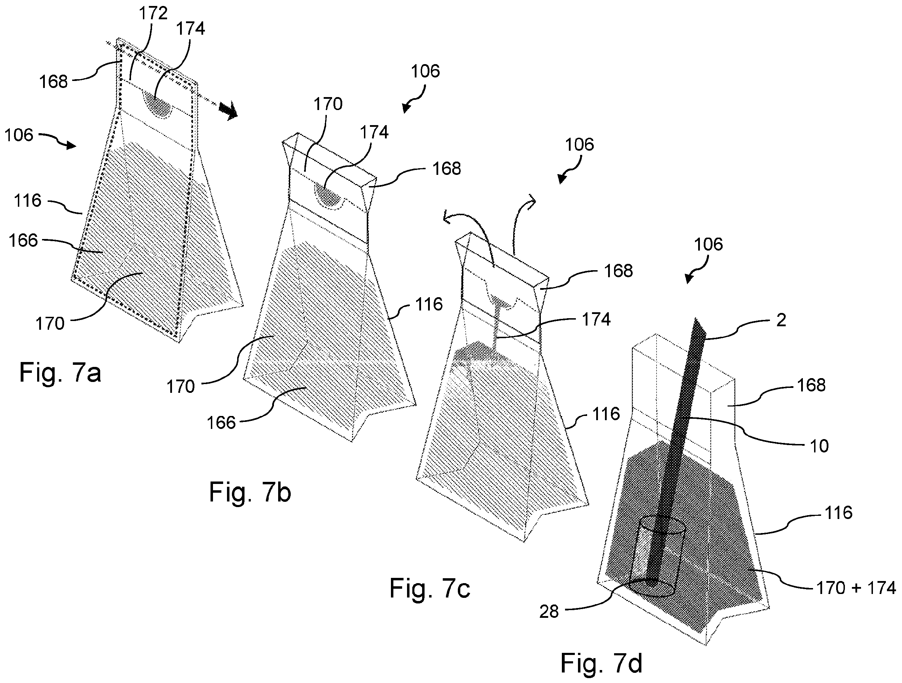

A cleaning apparatus 106 according to a preferred embodiment is shown in Figures 7a to 7d. The cleaning apparatus 106 comprises a bag 116 including a main body portion 166 and a neck portion 168 extending upwardly from the main body portion 166. Sides and a base of the bag are sealed to form an internal volume of the bag 116 holding a first volume of sterile or distilled water 170. A shaped frangible seal 172 extends across the neck portion 168 to seal the main body portion 166 of the bag 116.

The shape of the frangible seal 172 includes a recess or concavity holding a second volume of concentrated detergent 174. To form the recess, in this embodiment the frangible seal 172 comprises a weld line that extends in a direction generally across the neck portion 168, with a central region of the weld line having a semi-circular shape that extends generally in a direction towards the base of the bag 116.

An upper end of the neck portion 168 is sealed to close the upper end of the neck portion 168 and retain the concentrated detergent in a region of the neck portion 168 between the upper seal and the frangible seal 170. The concentrated detergent is therefore held in the neck portion 168 of the bag 116 separated from the sterile water in the main body portion 166 of the bag 116.

A tear slot or cut-out may be provided at one side of the neck portion 168 proximate its upper end. This provides a weakened area of weld to allow a user to subsequently tear open the neck portion 168 of the bag 116 at that point. It will be understood that the tear slot is disposed below the upper seal but above the frangible seal 170 and the volume of concentrated detergent.

In preferred embodiments, the bag contains 500ml of sterile water and the reservoir contains 5ml of concentrated detergent.

### Method

To carry out the cleaning method using the system of the present invention, the cleaning adaptor 102, detergent delivery assembly 104 and cleaning apparatus 106 are connected to the endoscope 2 with the light source connector 4 of the endoscope 2 still connected to the air pump, but with the air pump switched off.

The cleaning adaptor 102 is inserted into the air/water port 36 and engaged with the suction port 30 to seal the openings 56, 44 of each of the air/water port 36 and suction port 30 respectively.

The sealing plug 108 is pushed into the air/water port 36 so that the tubular stem 118 extends into the air/water cylinder 54 and the head portion 127 contacts the collar 58 surrounding the opening 56 of the air/water port 36. The sealing cap 110 is engaged with the suction port 30 by inserting the plug portion 134 into the suction cylinder 42 so that the sealing surface 136 contacts the collar 48 surrounding the opening 44 of the suction port 30. In some embodiments the annular retainer 138 may engage with a circumferential lip or undercut edge of the collar to retain the sealing cap 110 is engagement with the suction port 30.

With the sealing plug 108 inserted fully into the air/water cylinder 54, the annular seals 128 contact and seal against the internal surface of the wall of the air/water cylinder 54. A first one of the annular seals 128 at the first end 122 of the stem 118 is disposed between the air inlet 60 and the water inlet 64 and between the air outlet 62 and the water outlet 66. A second one of the annular seals 128 is disposed proximate the apertures 132 of the transverse passage 130, between the apertures 132 and the first annular seal 128. In use, this second annular seal 128 is located such that the air inlet 60 is disposed between the first annular seal 128 and the second annular seal 128. A third one of the annular seals 128 is disposed proximate the apertures 132 of the transverse passage 130, between the apertures 132 and the second end of the stem 118. In use, this third annular seal 128 is located such that the air outlet 62 is disposed between the second annular seal 128 and the third annular seal 128. It will be appreciated that the apertures 132 of the transverse passage 130 are also disposed between the second annular seal 128 and the third annular seal 128. In this way a fluid flow path is formed between the transverse passage 130 and the air outlet 62.

The detergent delivery assembly 104 is engaged with the water bottle connector 16 of the light source connector 4 of the endoscope 2 so that the air inlet conduit 150 is in fluid communication with the air channel 34 of the endoscope 2 and the liquid outlet conduit 152 is in fluid communication with the water channel 38 of the endoscope 2 through the water bottle connector 16.

When the detergent delivery assembly 104 is initially attached to the water bottle connector 16 the detergent concentrate is preferably still held in the detergent concentrate reservoir 158 separated from sterile water held in the pouch 112. Once connected, the break-to-open feature 160 may be broken to release the detergent concentrate into the sterile water to form a detergent solution within the pouch 112.

The distal tip 28 of the endoscope 2 is inserted into the bag 116 of the cleaning apparatus 106. To do this a user first tears or cuts open the upper end of the neck portion 168, below the upper seal and above the frangible seal 172, as shown in Figure 7a. Subsequently, by pulling on the sides of the neck portion 168 at its upper end to separate them (as illustrated in Figure 7b), a user can break the frangible seal 172 without tearing the walls of the bag 116. This dispenses the concentrated detergent 174 into the sterile water 170 as shown in Figure 7c. The detergent concentrate 174 mixes with the sterile water 170 in the main body 166 of the bag 116. The distal tip 28 of the endoscope 2 may then be inserted into the main body portion 166 of the bag 116 through the neck portion 168, as illustrated in Figure 7d.

Before securing the bag 116 to the insertion tube 10 of the endoscope 2, a user may first clean a tip section and the outer surfaces of the insertion tube 10 of the endoscope 2 with the detergent solution within the bag 116, with optional use of a suitable sponge.

In preferred embodiments, the neck portion 168 of the bag 116 includes a retainer for holding the tip 28 of the endoscope within the volume of detergent solution (170 + 174) in the bag 116. The retainer may be in the form of an elastic or sprung clip that grips a part of the insertion tube 10 of the endoscope 2. The retainer may, for example, be in the form of an elastically deformable ring engaged with the neck portion of the bag. The deformable ring may be biased into a position in which a narrow opening is provided in the neck portion and the deformable ring must be pressed or stretched to increase a dimension of the opening to allow the endoscope 2 to be inserted through the neck portion. With the force on the deformable ring released the ring returns to its original position and grips a part of the endoscope 2. Alternatively, the retainer may comprise a tie, for example a cable tie or similar. The retainer may comprise a first region of the neck portion 168 that may be adhered to a second region of the neck portion 168. One or both of the first and second regions may include a layer of adhesive. In use the first and second regions may be adhered to each other to effectively wrap the neck portion 168 around a part of the endoscope 2. The layer of adhesive may be exposed when a user opens the top of the bag 116 to use the cleaning apparatus 106.

Once all of the cleaning adaptor 102, detergent delivery assembly 104 and cleaning apparatus 106 have been connected to the endoscope 2, a user then switches on the air pump and the suction pump connected to the light source connector 4. Preferably the air pump and the suction pump are switched on simultaneously.

With the cleaning adaptor 102 sealing the air inlet 60 of the air/water cylinder 54 between the first annular seal 128 and the second annular seal 128, a flow of air from the air pump flows along the first section of the air channel 34 and the branch extending to the air port 18 of the water bottle connector 16. The air flows into the pouch 112 of the detergent delivery assembly 104 through the air inlet conduit 150 to pressurise the pouch 112. This forces detergent solution out of the pouch 112 through the liquid outlet conduit 152, through the water port 20 of the water bottle connector 16, and into the first section of the water channel 38 of the endoscope 2.

The detergent solution flows along the first section of the water channel 38 and into the air/water cylinder 54 through the water inlet 64. As there are no seals between the water inlet 64 and the water outlet 66, the detergent solution is free to flow out of the water outlet 66 and into the second section of the water channel 38.

The detergent solution is also able to flow through the opening 124 in the end surface 126 of the stem 118 and into the longitudinal passage 120. The liquid flows through the longitudinal passage 120, into the transverse passage 130, and out of the stem 118 through the apertures 132. As described above, the apertures 132 and the air outlet 62 are both disposed between the second and third annular seals 128 such that the detergent solution is able to flow through the air outlet 62 and into the second section of the air channel 34.

The detergent solution therefore flows along both the second section of the air channel 34 and the second section of the water channel 38 to flush the air/water channel 40 with detergent solution.

The detergent solution exits the air/water channel 40 through the distal tip 28 of the endoscope 2 and into the bag 116 of the cleaning apparatus 106.

Preferably the air/water channel 40 is flushed for 30 seconds in this way. If no detergent solution is drawn from the detergent delivery assembly 104 and/or if no detergent solution is seen entering the bag 116 of the cleaning apparatus 106 from the air/water channel 40, this indicates a blockage in the air channel 34 or water channel 38.

With the sealing cap 110 of the cleaning adaptor 102 sealing the opening 44 of the suction cylinder 42, the suction pump draws detergent from the bag 116 of the cleaning apparatus 106 through the suction channel 26 to flush the suction channel 26 with detergent solution. It will be understood that, to allow the detergent solution to flush through the length of the suction channel 26, the biopsy port 32 is covered or sealed to ensure the desired flow of detergent solution through the suction channel 26. A cover or seal provided at the biopsy port 32 preferably forms an air tight seal so that air is unable to flow into the suction/biopsy channel 26 through the biopsy port 32.

The detergent solution exits the suction channel 26 through the patient connecting tube (or similar tube) and into the suction jar (or other fluid collection vessel associated with the suction pump).

Preferably the suction channel 26 is flushed for 30 seconds in this way. If no detergent solution is drawn from the bag 116 of the cleaning apparatus 106 this indicates a blockage in the suction channel 26.

In preferred methods, before switching the suction pump and the air pump on, a volume of detergent solution may be drawn from the bag 116 of the cleaning apparatus 106 using a syringe or similar. The syringe may then be connected to a water jet channel or auxiliary water channel of the endoscope 2 to flush the water jet channel or auxiliary water channel with detergent solution. In particular the syringe may be connected to the water jet connection 24 of the light source connector 4. The detergent solution exits the water jet channel or auxiliary water channel through the distal tip 28 back into the bag 116 of the cleaning apparatus 106. It will be appreciated that other types of endoscope may include similar irrigation channels, working channels or instrument channels that may be flushed in a similar manner.

As described above, the air pump and suction pump are preferably operated for a pre-determined length of time to ensure that the air/water channel and suction channel are thoroughly flushed with the detergent solution. The time for which the channels are flushed may be dependent on the type and size of the endoscope. Larger diameter or longer endoscopes may require flushing for a longer length of time than smaller diameter or shorter endoscopes.

In some embodiments the system may include a processor that is configured to identify the type of endoscope that is connected to it. The processor may be configured to determine the length of time for which the air pump and suction pump are switched on to facilitate optimal cleaning. The processor may be configured to automatically switch off the air pump and the suction pump at the end of the pre-determined length of time. The air pump and the suction pump may be switched off at the same time or at different times.

Once flushing of the air/water channels 34, 38 and suction/biopsy channel 26 has been completed, the cleaning adaptor 102, detergent delivery assembly 104 and cleaning apparatus 106 are preferably left engaged with or connected to the endoscope 2. The light source connector 4 is then detached or disconnected from the air pump/light source, and the endoscope 2 is then covered for transportation to a reprocessing area, complete with the cleaning adaptor 102, detergent delivery assembly 104 and cleaning apparatus 106.

In preferred embodiments, the suction pump and air pump are switched off while all of the air/water channel, suction/biopsy channel and auxiliary water channel remain full of detergent solution along their complete length.

The advantage of the cleaning adaptor 102, detergent delivery assembly 104 and cleaning apparatus 106 remaining connected to the endoscope is that these elements can form part of a closed system that limits or prevents air flow through the air/water channels 34, 38 and suction channel 26, so that these channels 34, 38, 26 remain moist. This inhibits or prevents the development of biofilm in the air/water channels 34, 38 and suction/biopsy channel 26.

To achieve a fully closed system the syringe connected to the water jet channel or auxiliary water channel of the endoscope 2 preferably also remains attached, and in particularly preferred embodiments the syringe includes a non-return valve so that detergent solution cannot flow from the auxiliary water channel back into the syringe.

Similarly, a valve may be provided at the suction pump, or may be provided in the patient connecting tube (or similar tube between the light source connector and the suction pump). While the suction pump is drawing detergent solution through the suction channel 26, as described above, the connection of the light source connector is connected to a jar or container associated with the suction pump so that the detergent solution is able to flow through the valve and into the container. When the suction pump is switched off, before disconnection of the light source connector 4 from the air pump/light source, the valve in the tube or other part of the suction pump, is preferably configured to provide a seal such that detergent solution is unable to exit the suction channel at the light source connector. Furthermore, the valve is preferably configured to form an air tight seal so that air is unable to flow into the suction channel through the valve. It will be appreciated that the disconnection of the light source connector from the suction pump occurs in a position so that the valve remains connected to the light source connector. In a preferred embodiment the valve is provided in a patient connecting tube that connects the light source connector to the suction pump, and it is the end of the patient connecting tube that is released or disconnected from the suction pump so that the patient connecting tube remains attached to the light source connector.

With air tight seals provided at both the biopsy port 32 and the light source connector end of the suction channel 26, detergent solution in the suction channel 26 is unable to flow along the channel 26 and out of the tip 28 back into the cleaning apparatus 106 because air is unable to enter the channel 26 to replace it.

The cleaning apparatus 106 and syringe connected to the auxiliary water channel preferably form a similar air tight closed system along the auxiliary water channel to retain detergent solution in the auxiliary water channel.

The cleaning apparatus 106, cleaning adaptor 102 and detergent delivery assembly 104 are configured to form an air tight closed system to retain detergent solution along the length of the air and water channels 34, 38.

It will be understood that to retain the detergent solution in the channels 34, 38, 26 in this way, the distal tip 28 of the endoscope must remain submerged in a volume of detergent solution in the bag 116 of the cleaning apparatus 106. Once the flushing of the air/water channels 34, 38 and suction/biopsy channel 26 has been completed the endoscope 2 is generally then transported to a reprocessing area. Accordingly, it is preferable if the distal tip 28 of the endoscope 2 can be retained in a small but sufficient volume of detergent solution in such a way that the endoscope can be laid in a tray or similar for transportation with the tip 28 remaining submerged.

The cleaning apparatus 106 may therefore include a secondary securing system that enables the bag 116 to be more tightly secured around the distal end of the insertion tube 10 in such a way that the distal tip is held in a corner or lower section of the bag 116. Alternatively, the cleaning apparatus 106 may include a secondary container within the bag 116. The secondary container is filled with the detergent solution and is sized to receive the distal tip 28 of the insertion tube 10. Once the flushing procedure described above has been completed, the distal tip 28 may be moved from a primary internal volume of the bag 116 into the secondary container while remaining within the volume of detergent solution. The distal tip 28 with and in the secondary container may then be removed from the bag 116 for more easy transportation of the endoscope 2.

Before the next stage in the cleaning, sterilising and disinfection of the endoscope 2 the cleaning adaptor 102, detergent delivery assembly 104 and cleaning apparatus 106 are detached from the endoscope 2. Any other valves, covers syringes and the like may also be removed before sterilisation.

To remove the cleaning adaptor 102 from the air/water port 36 and suction port 30 of the endoscope 2 a user preferably pulls on the grip element 140 to disengage the sealing cap 110 from the collar 48 around the opening 44 of the suction port 30. The user can then continue to apply a pulling force to the grip element 140 to pull the sealing plug 108 out of the air/water cylinder 54. It will be appreciated that a reasonable force must be applied to the sealing plug 108 to overcome the frictional force between each of the annular seals 128 and the internal surface of the wall of the air/water cylinder 54.

As a user pulls upwardly (in a direction away from the control section 6 of the endoscope 2) on the grip element 140, this force is transferred to the head portion 127 of the sealing plug 108. The magnitude and direction of the force required to be applied to the head portion 127 to pull the stem 118 out of the air/water cylinder 54 is sufficient to break or rupture the weakened portion 146 of the stem 118. In this embodiment, the tensile force applied to the reduced thickness region 146 of the stem 118 is sufficient to tear the material in this region of the stem 118.

Once the weakened portion 146 of the stem 118 has been ruptured it will be appreciated that a fluid flow path is then formed from the longitudinal passage 120, through the ruptured portion and to an area external to the stem 118 between the head portion 127 and the annular seals 128. If the sealing plug 108 of the cleaning adaptor 102 is subsequently inserted into the air/water cylinder 54 of another endoscope 2, this fluid flow path through the ruptured portion will mean that the air/water port 36 can no longer be pressurised which prevents liquid being drawn up into the air/water cylinder 54.

All of the cleaning adaptor 102, detergent delivery assembly 104 and cleaning apparatus 106 are designed to be single use to minimise or prevent cross-contamination between endoscopes during cleaning and reprocessing.

While a single embodiment of each of the cleaning adaptor 102, detergent delivery assembly 104 and cleaning apparatus 106 has been described above, it will be appreciated that these elements of the system 100 may have any suitable configuration to enable them to function and be used as described above.

When used to clean medical equipment, such as an endoscope or similar, it is desirable to use a neutral pH enzymatic detergent solution. The detergent is designed to break down proteinaceous materials and prevent the formation of biofilm, for example in the air/water channel of the endoscope. Furthermore, it is preferable for the detergent solution to be of a relatively low concentration. It has been found, however, that pre-mixed low concentration detergent degrades over time. Tests show that bacterial growth occurs in low concentration detergent. Accordingly, if the detergent solution is stored for a low time before use, the detergent solution may have degraded to a point at which the detergent solution is no longer fit for purpose. Accordingly, it is desirable if the low concentration detergent solution is used soon after it is formed.

Advantageously, the detergent delivery assembly 104 includes a detergent concentrate reservoir 158 which keeps the detergent concentrate separate from the sterile water held in the pouch 112 until a user wishes to use the detergent delivery assembly 104. The detergent concentrate reservoir 158 may then be opened or ruptured to allow the detergent concentrate to mix with the sterile water to form the required lower concentration detergent solution shortly before use.

Similarly, the cleaning apparatus 106 preferably includes a seal or other feature to retain the concentrated detergent in the neck portion 168 of the bag 116 separated from the sterile water in the main body portion 166 of the bag 116 until a user wishes to use the cleaning apparatus 106. The bag 116 can then be opened to dispense or release the concentrated detergent into the sterile water to form the required lower concentration detergent solution shortly before use.

In the embodiment of the detergent delivery assembly 104 described above the closing of the first end of the air inlet conduit 150 by the detergent concentrate reservoir 158 and break-to-open feature 160 also means that it is not possible for the endoscope 2 to blow air into the pouch 112 to pressurise it until the end of the detergent concentrate reservoir 158 has been opened. Accordingly, this design of reservoir 158 provides a fail-safe solution that ensures that the pouch 112 can only be pressurised by air entering the pouch 112, and liquid can only flow out of the pouch 112, if the detergent concentrate reservoir 158 has been opened or ruptured.

### Detergent Delivery Assembly - Alternative Embodiments

In some embodiments of the detergent delivery assembly, the break-to-open feature at the end of the conduit used to seal the reservoir may be configured such that sufficient pressure within the air inlet conduit ruptures or otherwise breaks a part of the break-to-open feature. In these embodiments, in use, with the reservoir at the end of the air inlet conduit initially sealed, when the air pump is switched on the pressure in the air inlet conduit increases. When the pressure reaches a threshold pressure this, preferably, causes the break-to-open feature to rupture and forces the detergent into the sterile water. Continued activation of the air pump will then pressurise the pouch and force detergent solution through the liquid outlet conduit and into the air/water channels of the endoscope as described above.

In some embodiments a single break-off moulding is disposed at the first ends of both of the air inlet conduit and the liquid outlet conduit. This combined break-off moulding provides a cross contamination protection for the contents of the detergent reservoir (in the air inlet conduit) and the sterile water inside the bag or pouch. Breaking off the tab on the combined moulding opens the air inlet conduit and detergent concentrate reservoir, allowing air in to pressurise the pouch and releasing detergent concentrate into the sterile water. It also opens the liquid outlet conduit which allows the detergent solution to exit the pouch and enter the endoscope.

In some embodiments of the detergent delivery assembly at least one of the air and water ports of the connector may include a one-way valve arranged to prevent the flow of fluid from the pouch out of the connector when the valve is closed. Preferably the valve is a reed valve or duck bill valve.

In some embodiments of the detergent delivery assembly the detergent concentrate reservoir may be a receptacle in the form of a sachet or sealed tube that is attached to an edge or side seam of the pouch. The sachet may be positioned with respect to the pouch such that, once the reservoir sachet has been filled with detergent and the pouch has been filled with water, the sachet may be sealed concurrently with the pouch. In some examples the sachet is designed such that the sachet ruptures when pressure is applied to it. Accordingly, the material from which the sachet is formed may be thinner than the material from which the pouch is formed. Alternatively, the sachet may have a break-to-open feature.

In other examples the reservoir may be in the form of a separate small sachet that can be ruptured when pressure is applied to it. The sachet may be separate, i.e. not attached to the sides of the pouch, but retained within the internal volume of the pouch. The detergent concentrate reservoir could be in the form of a spherical 'bead' that is ruptured when it is squeezed.

At least a part of the detergent concentrate reservoir is preferably disposed below the surface of the sterile water within the pouch when the internal volume of the pouch is filled with sterile water.

### Cleaning Apparatus - Alternative Embodiments

In alternative embodiments of the cleaning apparatus 106, a reservoir for holding the volume of detergent concentrate may be in the form of a narrow tubular bag or pouch, rather than being formed by a frangible seal as described above. In these embodiments the tubular pouch preferably has a diameter small enough to sit inside the neck of the bag. During manufacture of the cleaning apparatus, the top of the smaller tubular pouch (reservoir) may be heat sealed at its top concurrently with a top of the neck of the bag. Heat sealing may therefore be used to seal both the reservoir and the bag, and may also attach the reservoir to the bag. In these embodiments the reservoir pouch may be attached to the bag so that the reservoir is suspended from a top edge of the neck portion of the bag and hangs down or extends into the main body portion of the bag. In this way, the concentrated detergent is contained within the reservoir in the neck of the larger bag isolated from the sterile water within the main internal volume of the bag. The heat seal is preferably disposed above a line across which a user is guided to tear or cut open the bag to gain access to the internal volume of the bag. Accordingly, when the user tears open the neck of the bag, the smaller reservoir (now detached from the bag) falls into the main body portion of the bag. The concentrated detergent is then mixed with the sterile water to form the lower concentration detergent solution.

In some embodiments, the reservoir may be attached to a side or base of the bag. In some examples the reservoir is designed such that the reservoir ruptures when pressure is applied to it. Accordingly, the material from which the reservoir is formed may be thinner than the material from which the bag is formed. Alternatively, the reservoir may have a break-to-open feature. In other examples the reservoir may be in the form of a separate small sachet that can be ruptured when pressure is applied to it. The sachet may be separate, i.e. not attached to the sides, base or neck of the bag, but may be retained within the internal volume of the bag, for example within the volume of sterile water. The detergent concentrate reservoir may be in the form of a spherical 'bead' that is ruptured when it is squeezed.

In some cases it may be desirable to protect the distal tip 28 of the endoscope 2 with a tip protector (such as that disclosed in EP3206555) during cleaning and reprocessing of the endoscope, to prevent the distal tip being knocked and damaged. Accordingly, in preferred embodiments of the cleaning apparatus the neck of the bag has an internal diameter large enough to allow the tip portion of the endoscope with the tip protector attached to be inserted into the bag and removed from the bag through the neck without the tip protector being pulled off the end of the endoscope 2.

In some applications, a cylindrical sponge may be used to wipe detergent onto the endoscope 2, in particular onto external surfaces of the insertion tube of the endoscope 2. In some examples the cylindrical sponge has a hole through the centre and a slit in one side and is designed to fit around the endoscope insertion tube 10. In use, a user slides the sponge along the endoscope insertion tube 10 and agitates it back and forth to clean the endoscope 2. In these cases, the neck of the bag of the cleaning apparatus preferably has dimensions large enough to accommodate this sponge so that a user may use the detergent solution within the bag to clean external surfaces of the endoscope. Typically, this type of sponge may be 50mm in diameter and 100mm long.

As described above, in some applications a cylindrical sponge may be used to wipe detergent onto the endoscope. The cylindrical sponge may have a hole or bore through the centre and a slit in one side such that it fits around the endoscope insertion tube. In these cases, the retainer may be configured to grip or clamp the sponge around the endoscope to retain the tip of the endoscope in the bag. This has the advantage that the sponge provides a layer of protection between the retainer and the insertion tube of the endoscope.

### Cleaning Adaptor - Alternative Embodiments

Although in the above embodiment the cleaning adaptor 102 included both a sealing plug 108 for insertion into the air/water cylinder 54 of the endoscope 2 and a sealing cap 110 for sealing the suction port 30 of the endoscope 2, in other embodiments the cleaning adaptor may only include a sealing plug and not a sealing cap. As discussed above the air/water channel and the suction channel are flushed in separate procedures. Accordingly, the cleaning adaptor may only include a sealing plug for use during flushing of the air/water channels. A sealing cap for the suction port 30 may be provided separately from the cleaning adaptor.

In these embodiments, a grip element or grip portion may protrude from an upper surface of the head portion of the sealing plug. The grip element may be gripped by a user to both push or insert the stem of the sealing plug into the air/water cylinder and pull the stem of the sealing plug out of the air/water cylinder. Pulling on the grip element may still cause a weakened portion of the stem to rupture.

It will be appreciated that the grip element may be of any suitable form to allow a user to apply sufficient force to the cleaning adaptor to both withdraw the sealing plug from the air/water cylinder and rupture the weakened portion. In some embodiments the grip element may be provided by a tab that extends from an edge of a part of the cleaning adaptor. For example, the tab may extend from a circumferential edge of the sealing cap.

In further embodiments the cleaning adaptor comprises a sealing plug for insertion into the air/water cylinder of the air/water port of the endoscope, a sealing cap for engagement with and sealing of the suction port of the endoscope, and a flow modifying stem extending from the sealing cap into the suction port. The flow modifying stem preferably has a generally cylindrical outer surface including a spiral channel or helical groove extending along the length of the flow modifying stem. The outer surface of the flow modifying stem may also comprise a deflection surface. In some embodiments the flow modifying stem may be rotatable. This rotation may be driven by a flow of fluid around the flow modifying stem.

During flushing of the suction channel with the detergent solution, as the solution enters the suction cylinder at least a part of the volume of the liquid strikes the flow modifying stem. The helical groove in the outer surface causes the liquid to flow around the flow modifying stem thereby creating a vortex flow. This more turbulent flow in the suction cylinder increases the ability of the liquid to clean or scour the inner surfaces of the suction cylinder. The turbulence of the flow of the liquid may be increased further by providing a textured outer surface of the flow modifying stem, including surfaces of the helical groove.

While in the above embodiment the flow modifying stem included a helical groove in an outer surface, in other embodiments the stem may not include such a groove. The presence of a cylindrical stem within the suction cylinder may be sufficient to create more turbulent fluid flow within the suction cylinder and/or to force the flow of fluid in the suction cylinder generally towards or against the inner surfaces of the suction cylinder. In other embodiments the outer surface of the generally cylindrical stem may be textured to provide a more turbulent fluid flow within the suction cylinder. In further embodiments the flow modifying stem may comprise fins or other protrusions extending radially outwardly from a central rod or shaft. The fins or protrusions may have a helical arrangement.

While in the above embodiment a syringe filled with detergent solution was connected to the water jet channel or auxiliary water channel of the endoscope, in other embodiments a capsule or container pre-filled with detergent solution may be connected to the water jet channel or auxiliary water channel. The pre-filled container may be similar to the detergent delivery assembly described above.

Other modifications and variations not explicitly disclosed above may also be contemplated without departing from the scope of the invention as defined in the appended claims.

## Claims

1. A kit for flushing internal channels of an endoscope (2), the kit comprising:
a sealing plug (108) for engagement with an air/water cylinder (54) of an endoscope (2), and the sealing plug (108) being configured, in use, to form a seal at an air inlet (60) of the air/water cylinder (54) and to permit fluid flow between a water inlet (64) of the air/water cylinder (54) and both an air outlet (62) and a water outlet (66) of the air/water cylinder (54) simultaneously; **characterized in that** the kit further comprises
a detergent delivery assembly (104) for connection to a water bottle connector (16) of the endoscope (2), the detergent delivery assembly (104) comprising a bag (112) holding a volume of detergent solution, a first fluid conduit (150) extending between a first end disposed within the bag (112) and a second end external to the bag (112) for connection to an air port of the water bottle connector (16), and a second fluid conduit (152) extending between a first end disposed within the bag (112) and a second end external to the bag (112) for connection to a water port of the water bottle connector (16); and
a cleaning apparatus (106) comprising a bag (116) holding a volume of detergent solution, the bag (116) having a neck portion (168) for insertion of a distal tip (28) of the endoscope (2) into the bag (116).

2. A kit according to Claim 1, in which the sealing plug (108) is a unitary element having no moving parts.

3. A kit according to Claim 1 or Claim 2, further comprising a sealing cap (110) for sealing a suction port (30) of the endoscope (2).

4. A kit according to any preceding claim, in which the sealing plug (108) comprises:
a tubular stem (118) extending between first and second ends and including a longitudinal passage (120), the longitudinal passage (120) being open at the first end of said stem (118);
a head portion (127) closing the longitudinal passage (120) at the second end of said stem (118) and the head portion (127) including a flange extending radially outwardly from the tubular stem (118);
at least three annular seals (128) extending radially outwardly from the tubular stem (118) and being spaced apart along a length of the tubular stem (118); and
a transverse passage (130) extending through the tubular stem (118) and providing a fluid flow path between the longitudinal passage (120) and an aperture (132) in an outer surface of the tubular stem (118), the aperture (132) being disposed between two of the at least three annular seals (128).

5. A kit according to any preceding claim, in which the detergent delivery assembly (104) further comprises a rupturable or breakable reservoir (158) within an internal volume of the bag (112), the reservoir (158) holding a volume of a detergent concentrate and the internal volume of the bag (112) holding a volume of water.

6. A kit according to any preceding claim, in which the bag (116) of the cleaning apparatus (106) comprises a main body portion (166) providing an internal volume for holding a volume of water, and the cleaning apparatus (106) further comprises a reservoir holding a volume of detergent concentrate, the reservoir being engaged with the neck portion (168) of the bag such that, in use, when the neck portion (168) is opened to access the internal volume of the main body portion (166), the detergent concentrate is concurrently released from the reservoir to mix with the water.

7. A system for flushing internal channels of an endoscope (2) comprising:
a sealing plug (108) engaged with an air/water cylinder (54) of the endoscope (2), the sealing plug (108) forming a seal at an air inlet (60) of the air/water cylinder (54) while permitting fluid flow between a water inlet (64) of the air/water cylinder (54) and both an air outlet (62) and a water outlet (66) of the air/water cylinder (54) simultaneously; **characterized in that** the system further comprises
a detergent delivery assembly (104) connected to a water bottle connector (16) of the endoscope (2), the detergent delivery assembly (104) comprising a bag (112) holding a volume of detergent solution, a first fluid conduit (150) extending between a first end disposed within the bag (112) and a second end in fluid connection with an air port of the water bottle connector (16), and a second fluid conduit (152) extending between a first end disposed within the bag (112) and a second end in fluid connection with a water port of the water bottle connector (16); and
a cleaning apparatus (106) comprising a bag (116) holding a volume of detergent solution, a distal tip (28) of the endoscope (2) being submerged in the volume of detergent solution in the bag (116).

8. A system according to Claim 7, further comprising a sealing cap (110) engaged with and sealing a suction port (30) of the endoscope (2).

9. A system according to Claim 8, in which the sealing plug (108) and the sealing cap (110) are parts of a unitary cleaning adaptor (102) made of a resilient polymeric material.

10. A system according to any one of Claims 7 to 9, in which a retainer is secured around a part of the endoscope (2) to secure the cleaning apparatus (106) to the endoscope (2) such that the distal tip (28) remains submerged during use.

11. A method of flushing internal channels of an endoscope (2) using a kit according to any one of Claims 1 to 6, the method comprising:
keeping a light source connector of the endoscope (2) comprising a connector (14) for connection to an air pump connected to an air pump with the air pump initially switched off;
engaging the sealing plug (108) with an air/water cylinder (54) of the endoscope (2) so that a seal is formed at an air inlet (60) of the air/water cylinder (54), while permitting fluid flow from a water inlet (64) of the air/water cylinder (54) to both an air outlet (62) and a water outlet (66) of the air/water cylinder (54) simultaneously;
connecting the detergent delivery assembly (104) to a water bottle connector (16) of the endoscope (2);
inserting a distal tip (28) of the endoscope (2) into the bag (116) of the cleaning apparatus (106) so that the distal tip (28) is submerged in the volume of detergent solution; and
switching on the air pump so that detergent solution from the detergent delivery assembly (104) is forced through an air channel (34) and a water channel (38) of the endoscope (2).

12. A method according to Claim 11, further comprising:
sealing a suction port (30) of the endoscope (2) with a sealing cap (110); and
switching on a suction pump connected to the light source connector so that detergent solution from the cleaning apparatus (106) is drawn through a suction channel (26) of the endoscope (2).

13. A method according to Claim 11 or Claim 12, further comprising, after connecting the detergent delivery assembly (104) to the water bottle connector (16) and before switching on the air pump, rupturing or breaking a reservoir (158) within an internal volume of the bag (112) of the detergent delivery assembly (104) so that detergent concentrate held within the reservoir (158) mixes with water in the internal volume of the bag (112).

14. A method according to any one of Claims 11 to 13 using a kit according to Claim 6, further comprising, before inserting the distal tip (28) of the endoscope (2), opening the neck portion (168) of the bag (116) of the cleaning apparatus (106) to access the internal volume of the main body portion (166) and simultaneously releasing detergent concentrate from the reservoir to mix with the water in the main body portion (166).

15. A method according to any one of Claims 11 to 14, further comprising:
switching off the air pump;
disconnecting the light source connector from the air pump; and
covering the endoscope (2) for transportation,
wherein, the sealing plug (108), detergent delivery assembly (104) and cleaning apparatus (106) remain connected to the endoscope (2) when it is covered.

## Patentansprüche

1. Set zum Spülen der Innenkanäle eines Endoskops (2), wobei das Set folgendes umfasst:
einen Dichtungsstopfen (108) zum Eingriff mit einem Luft-/Wasserzylinder (54) eines Endoskops (2), wobei der Dichtungsstopfen (108) so konfiguriert ist, dass er im Gebrauch eine Dichtung an einem Lufteinlass (60) des Luft-/Wasserzylinders (54) bildet und gleichzeitig eine Fluidströmung zwischen einem Wassereinlass (64) des Luft-/Wasserzylinders (54) und sowohl einem Luftauslass (62) als auch einem Wasserauslass (66) des Luft-/Wasserzylinders (54) ermöglicht;
**dadurch gekennzeichnet,**
**dass** das Set ferner folgendes umfasst:
eine Reinigungsmittelzuführanordnung (104) zum Anschluss an einen Wasserflaschenanschluss (16) des Endoskops (2), wobei die
Reinigungsmittelzuführanordnung (104) einen Beutel (112) umfasst, der ein Volumen an Reinigungsmittellösung enthält,
eine erste Fluidleitung (150), die sich zwischen einem ersten Ende, das innerhalb des Beutels (112) angeordnet ist, und einem zweiten Ende außerhalb des Beutels (112) erstreckt, und zum Anschluss an einen Luftanschluss des Wasserflaschenanschlusses (16) vorgesehen ist, und
eine zweite Fluidleitung (152), die sich zwischen einem ersten Ende innerhalb des Beutels (112) und einem zweiten Ende außerhalb des Beutels (112) erstreckt und zum Anschluss an einen Wasseranschluss des Wasserflaschen-anschlusses (16) vorgesehen ist; und
eine Reinigungsvorrichtung (106), die einen Beutel (116) umfasst, der ein Volumen an Reinigungslösung enthält, wobei der Beutel (116) einen Halsabschnitt (168) zum Einführen einer distalen Spitze (28) des Endoskops (2) in den Beutel (116) aufweist.

2. Set nach Anspruch 1,
bei welchem der Verschlussstopfen (108) ein einteiliges Element ohne bewegliche Teile ist.

3. Set nach Anspruch 1 oder Anspruch 2,
welches zusätzlich eine Verschlusskappe (110) zum Verschließen eines Sauganschlusses (30) des Endoskops (2) umfasst.

4. Set nach einem der vorigen Ansprüche,
bei welchem der Verschlussstopfen (108) folgendes umfasst:
einen röhrenförmigen Schaft (118), der sich zwischen einem ersten und einem zweiten Ende erstreckt und einen Längsdurchgang (120) aufweist, wobei der Längsdurchgang (120) am ersten Ende des Schafts (118) offen ist;
einen Kopfabschnitt (127), der den Längsdurchgang (120) am zweiten Ende des Schafts (118) verschließt, wobei der Kopfabschnitt (127) einen Flansch umfasst, der sich vom röhrenförmigen Schaft (118) aus radial nach außen erstreckt;
wenigstens drei ringförmige Dichtungen (128), die sich vom röhrenförmigen Schaft (118) aus radial nach außen erstrecken und entlang der Länge des röhrenförmigen Schafts (118) voneinander beabstandet sind; und
einen Querkanal (130), der sich durch den röhrenförmigen Schaft (118) erstreckt und einen Fluidströmungsweg zwischen dem Längskanal (120) und einer Öffnung (132) in einer Außenfläche des röhrenförmigen Schafts (118) ausbildet, wobei die Öffnung (132) zwischen zwei der wenigstens drei ringförmigen Dichtungen (128) angeordnet ist.

5. Set nach einem der vorigen Ansprüche,
bei welchem die Reinigungsmittelzuführanordnung (104) ferner einen aufbrechbaren oder zerbrechbaren Vorratsbehälter (158) innerhalb eines Innenvolumens des Beutels (112) umfasst, wobei der Vorratsbehälter (158) ein Volumen eines Reinigungsmittelkonzentrats enthält und das Innenvolumen des Beutels (112) ein Volumen an Wasser enthält.

6. Set nach einem der vorigen Ansprüche,
wobei der Beutel (116) der Reinigungsvorrichtung (106) einen Hauptkörperabschnitt (166) umfasst, der ein Innenvolumen zur Aufnahme eines Volumens an Wasser bereitstellt, und die Reinigungsvorrichtung (106) ferner einen Vorratsbehälter umfasst, der ein Volumen an Waschmittelkonzentrat aufnimmt, wobei der Vorratsbehälter mit dem Halsabschnitt (168) des Beutels in Eingriff steht, so dass im Gebrauchszustand, wenn der Halsabschnitt (168) für den Zugang zum Innenvolumen des Hauptkörperabschnitts (166) geöffnet wird, gleichzeitig das Reinigungsmittelkonzentrat aus dem Vorratsbehälter freigesetzt wird, damit es sich mit dem Wasser vermischt.

7. System zum Spülen der Innenkanäle eines Endoskops (2), umfassend:
einen Dichtungsstopfen (108), der mit einem Luft-/Wasserzylinder (54) des Endoskops (2) in Eingriff steht, wobei der Dichtungsstopfen (108) eine Dichtung an einem Lufteinlass (60) des Luft-/Wasserzylinders (54) bildet, während er gleichzeitig einen Fluidströmung zwischen einem Wassereinlass (64) des Luft-/Wasserzylinders (54) und sowohl einem Luftauslass (62) als auch einem Wasserauslass (66) des Luft-/Wasserzylinders (54) ermöglicht;
**dadurch gekennzeichnet,**
**dass** das System ferner folgendes umfasst:
eine Reinigungsmittelzuführanordnung (104), die mit einem Wasserflaschenanschluss (16) des Endoskops (2) verbunden ist, wobei die Reinigungsmittelzuführanordnung (104) einen Beutel (112) umfasst, der ein Volumen an Reinigungsmittellösung enthält,
eine erste Fluidleitung (150), die sich zwischen einem ersten Ende, das innerhalb des Beutels (112) angeordnet ist, und einem zweiten Ende erstreckt, das in Fluidverbindung mit einem Luftanschluss des Wasserflaschenanschlusses (16) steht, und
eine zweite Fluidleitung (152), die sich zwischen einem ersten Ende, das innerhalb des Beutels (112) angeordnet ist, und einem zweiten Ende erstreckt, das in Fluidverbindung mit einem Wasseranschluss des Wasserflaschenanschlusses (16) steht;
und eine Reinigungsvorrichtung (106), die einen Beutel (116) umfasst, der ein Volumen an Reinigungslösung enthält, wobei eine distale Spitze (28) des Endoskops (2) in das Volumen der Reinigungslösung im Beutel (116) eingetaucht ist.

8. System nach Anspruch 7,
welches ferner eine Verschlusskappe (110) umfasst, die mit einem Sauganschluss (30) des Endoskops (2) in Eingriff steht und diesen abdichtet.

9. System nach Anspruch 8,
bei welchem der Verschlussstopfen (108) und die Verschlusskappe (110) Teile eines einheitlichen Reinigungsadapters (102) aus einem elastischen Polymermaterial sind.

10. System nach einem der Ansprüche 7 bis 9,
bei welchem eine Haltevorrichtung um einen Teil des Endoskops (2) herum befestigt ist, um die Reinigungsvorrichtung (106) so am Endoskop (2) zu befestigen, dass die distale Spitze (28) während ihrer Verwendung eingetaucht bleibt.

11. Verfahren zum Spülen der Innenkanäle eines Endoskops (2) unter Verwendung eines Sets nach einem der Ansprüche 1 bis 6, wobei das Verfahren folgendes umfasst:
In Verbindung Halten eines Lichtquellenanschlusses des Endoskops (2), welcher einen Anschluss (14) zum Anschluss an eine Luftpumpe umfasst, mit einer Luftpumpe, wobei die Luftpumpe zunächst ausgeschaltet ist;
in Eingriff Bringen des Dichtungsstopfens (108) in einen Luft-/Wasserzylinder (54) des Endoskops (2), so dass eine Dichtung an einem Lufteinlass (60) des Luft-/Wasserzylinders (54) gebildet wird, während gleichzeitig ein Fluidströmung von einem Wassereinlass (64) des Luft-/Wasserzylinders (54) sowohl zu einem Luftauslass (62) als auch zu einem Wasserauslass (66) des Luft-/ Wasserzylinders (54) ermöglicht wird;
Verbinden der Reinigungsmittelzuführanordnung (104) mit einem Wasserflaschenanschluss (16) des Endoskops (2);
Einführen einer distalen Spitze (28) des Endoskops (2) in den Beutel (116) der Reinigungsvorrichtung (106), so dass die distale Spitze (28) in das Volumen der Reinigungsmittellösung eingetaucht ist; und
Einschalten der Luftpumpe, so dass Reinigungsmittellösung aus der Reinigungsmittelzuführanordnung (104) durch einen Luftkanal (34) und einen Wasserkanal (38) des Endoskops (2) gezwungen wird.

12. Verfahren nach Anspruch 11,
welches ferner folgendes umfasst:
Verschließen einer Saugöffnung (30) des Endoskops (2) mit einer Verschlusskappe (110); und
Einschalten einer mit dem Lichtquellenanschluss verbundenen Saugpumpe, so dass Reinigungslösung aus der Reinigungsvorrichtung (106) durch einen Saugkanal (26) des Endoskops (2) gesaugt wird.

13. Verfahren nach Anspruch 11 oder Anspruch 12, welches ferner umfasst, dass nach dem Verbinden der Reinigungsmittelzuführanordnung (104) mit dem Wasserflaschenanschluss (16) und vor dem Einschalten der Luftpumpe ein Vorratsbehälter (158) innerhalb eines Innenvolumens des Beutels (112) der Reinigungsmittelzuführanordnung (104) aufgebrochen oder zerbrochen wird, so dass das in dem Vorratsbehälter (158) enthaltene Reinigungsmittelkonzentrat (158) mit Wasser im Innenraum des Beutels (112) vermischt wird.

14. Verfahren nach einem der Ansprüche 11 bis 13 unter Verwendung eines Sets nach Anspruch 6, welches ferner umfasst, dass vor dem Einführen der distalen Spitze (28) des Endoskops (2) das Öffnen des Halsabschnitts (168) des Beutels (116) der Reinigungsvorrichtung (106) erfolgt, um Zugang zum Innenvolumen des Hauptkörperabschnitts (166) zu erhalten, und gleichzeitig Freisetzen von Waschmittelkonzentrat aus dem Vorratsbehälter erfolgt, um es mit dem Wasser im Hauptkörperabschnitt (166) zu vermischen.

15. Verfahren nach einem der Ansprüche 11 bis 14,
das ferner folgendes umfasst:
Ausschalten der Luftpumpe;
Trennen des Lichtquellenanschlusses von der Luftpumpe; und
Abdecken des Endoskops (2) für den Transport, wobei der Verschlussstopfen (108), die Reinigungsmittelzuführanordnung (104) und die Reinigungsvorrichtung (106) mit dem Endoskop (2) verbunden bleiben, wenn es abgedeckt ist.

## Revendications

1. - Kit de rinçage de canaux internes d'un endoscope (2), le kit comprenant :
un bouchon d'étanchéité (108) destiné à venir en prise avec un cylindre air/eau (54) d'un endoscope (2), et le bouchon d'étanchéité (108) étant configuré, en cours d'utilisation, pour former un joint d'étanchéité à une entrée d'air (60) du cylindre air/eau (54) et pour permettre un écoulement de fluide entre une entrée d'eau (64) du cylindre air/eau (54) et à la fois une sortie d'air (62) et une sortie d'eau (66) du cylindre air/eau (54) de manière simultanée ;
**caractérisé par le fait que** le kit comprend en outre :
un ensemble de distribution de détergent (104) destiné à être relié à un raccord de bouteille d'eau (16) de l'endoscope (2), l'ensemble de distribution de détergent (104) comprenant un sac (112) contenant un volume de solution de détergent, un premier conduit de fluide (150) s'étendant entre une première extrémité disposée à l'intérieur du sac (112) et une seconde extrémité externe au sac (112) pour une liaison à un orifice d'air du raccord de bouteille d'eau (16), et un second conduit de fluide (152) s'étendant entre une première extrémité disposée à l'intérieur du sac (112) et une seconde extrémité externe au sac (112) pour une liaison à un orifice d'eau du raccord de bouteille d'eau (16) ; et
un appareil de nettoyage (106) comprenant un sac (116) contenant un volume de solution de détergent, le sac (116) ayant une partie col (168) pour l'introduction d'une pointe distale (28) de l'endoscope (2) dans le sac (116).

2. - Kit selon la revendication 1, dans lequel le bouchon d'étanchéité (108) est un élément unitaire ne comportant pas de pièces mobiles.

3. - Kit selon la revendication 1 ou la revendication 2, comprenant en outre un capuchon d'étanchéité (110) pour le scellement étanche d'un orifice d'aspiration (30) de l'endoscope (2).

4. - Kit selon l'une quelconque des revendications précédentes, dans lequel le bouchon d'étanchéité (108) comprend :
une tige tubulaire (118) s'étendant entre une première et une seconde extrémité et comprenant un passage longitudinal (120), le passage longitudinal (120) étant ouvert à la première extrémité de ladite tige (118) ;
une partie tête (127) fermant le passage longitudinal (120) à la seconde extrémité de ladite tige (118) et la partie tête (127) comprenant une bride s'étendant radialement vers l'extérieur à partir de la tige tubulaire (118) ;
au moins trois joints d'étanchéité annulaires (128) s'étendant radialement vers l'extérieur à partir de la tige tubulaire (118) et qui sont espacés le long d'une longueur de la tige tubulaire (118) ; et
un passage transversal (130) s'étendant à travers la tige tubulaire (118) et fournissant un trajet d'écoulement de fluide entre le passage longitudinal (120) et une ouverture (132) dans une surface externe de la tige tubulaire (118), l'ouverture (132) étant disposée entre deux des au moins trois joints d'étanchéité annulaires (128).

5. - Kit selon l'une quelconque des revendications précédentes, dans lequel l'ensemble de distribution de détergent (104) comprend en outre un réservoir apte à être rompu ou cassé (158) dans un volume interne du sac (112), le réservoir (158) contenant un volume d'un concentré de détergent et le volume interne du sac (112) contenant un volume d'eau.

6. - Kit selon l'une quelconque des revendications précédentes, dans lequel le sac (116) de l'appareil de nettoyage (106) comprend une partie corps principal (166) fournissant un volume interne pour contenir un volume d'eau, et l'appareil de nettoyage (106) comprend en outre un réservoir contenant un volume de concentré de détergent, le réservoir étant en prise avec la partie col (168) du sac de telle sorte qu'en cours d'utilisation, lorsque la partie col (168) est ouverte pour accéder au volume interne de la partie corps principal (166), le concentré de détergent est simultanément libéré du réservoir pour se mélanger à l'eau.

7. - Système de rinçage de canaux internes d'un endoscope (2), comprenant :
un bouchon d'étanchéité (108) en prise avec un cylindre air/eau (54) de l'endoscope (2), le bouchon d'étanchéité (108) formant un joint d'étanchéité à une entrée d'air (60) du cylindre air/eau (54) tout en permettant un écoulement de fluide entre une entrée d'eau (64) du cylindre air/eau (54) et à la fois une sortie d'air (62) et une sortie d'eau (66) du cylindre air/eau (54) de manière simultanée ;
**caractérisé par le fait que** le système comprend en outre :
un ensemble de distribution de détergent (104) relié à un raccord de bouteille d'eau (16) de l'endoscope (2), l'ensemble de distribution de détergent (104) comprenant un sac (112) contenant un volume de solution de détergent, un premier conduit de fluide (150) s'étendant entre une première extrémité disposée à l'intérieur du sac (112) et une seconde extrémité en liaison fluidique avec un orifice d'air du raccord de bouteille d'eau (16), et un second conduit de fluide (152) s'étendant entre une première extrémité disposée à l'intérieur du sac (112) et une seconde extrémité en liaison fluidique avec un orifice d'eau du raccord de bouteille d'eau (16) ; et
un appareil de nettoyage (106) comprenant un sac (116) contenant un volume de solution de détergent, une pointe distale (28) de l'endoscope (2) étant plongée dans le volume de solution de détergent dans le sac (116).

8. - Système selon la revendication 7, comprenant en outre un capuchon d'étanchéité (110) en prise avec et scellant de manière étanche un orifice d'aspiration (30) de l'endoscope (2).

9. - Système selon la revendication 8, dans lequel le bouchon d'étanchéité (108) et le capuchon d'étanchéité (110) font partie d'un adaptateur de nettoyage unitaire (102) réalisé en matériau polymère souple.

10. - Système selon l'une quelconque des revendications 7 à 9, dans lequel un dispositif de retenue est fixé autour d'une partie de l'endoscope (2) pour fixer l'appareil de nettoyage (106) à l'endoscope (2) de telle sorte que la pointe distale (28) reste plongée pendant l'utilisation.

11. - Procédé de rinçage de canaux internes d'un endoscope (2) à l'aide d'un kit selon l'une quelconque des revendications 1 à 6, le procédé comprenant :
maintenir un connecteur de source lumineuse de l'endoscope (2) comprenant un raccord (14) destiné à être relié à une pompe à air, relié à une pompe à air avec la pompe à air initialement à l'arrêt ;
mettre en prise le bouchon d'étanchéité (108) avec un cylindre air/eau (54) de l'endoscope (2) de manière à former un joint d'étanchéité à une entrée d'air (60) du cylindre air/eau (54), tout en permettant un écoulement de fluide d'une entrée d'eau (64) du cylindre air/eau (54) à la fois à une sortie d'air (62) et à une sortie d'eau (66) du cylindre air/eau (54) de manière simultanée ;
relier l'ensemble de distribution de détergent (104) à un raccord de bouteille d'eau (16) de l'endoscope (2) ;
introduire une pointe distale (28) de l'endoscope (2) dans le sac (116) de l'appareil de nettoyage (106) de telle sorte que la pointe distale (28) est plongée dans le volume de solution de détergent ; et
mettre en marche la pompe à air de telle sorte que la solution de détergent provenant de l'ensemble de distribution de détergent (104) est forcée à travers un canal d'air (34) et un canal d'eau (38) de l'endoscope (2).

12. - Procédé selon la revendication 11, comprenant en outre :
sceller de manière étanche un orifice d'aspiration (30) de l'endoscope (2) à l'aide d'un capuchon d'étanchéité (110) ; et
mettre en marche une pompe d'aspiration reliée au connecteur de source lumineuse afin que la solution de détergent provenant de l'appareil de nettoyage (106) soit aspirée à travers un canal d'aspiration (26) de l'endoscope (2).

13. - Procédé selon la revendication 11 ou la revendication 12, comprenant en outre, après avoir relié l'ensemble de distribution de détergent (104) au raccord de bouteille d'eau (16) et avant de mettre en marche la pompe à air, la rupture ou la cassure d'un réservoir (158) dans un volume interne du sac (112) de l'ensemble de distribution de détergent (104) de telle sorte qu'un concentré de détergent contenu dans le réservoir (158) se mélange à l'eau dans le volume interne du sac (112).

14. - Procédé selon l'une quelconque des revendications 11 à 13 en utilisant un kit selon la revendication 6, comprenant en outre, avant d'introduire la pointe distale (28) de l'endoscope (2), l'ouverture de la partie col (168) du sac (116) de l'appareil de nettoyage (106) pour accéder au volume interne de la partie corps principal (166) et la libération simultanée du concentré de détergent à partir du réservoir pour se mélanger à l'eau dans la partie corps principal (166).

15. - Procédé selon l'une quelconque des revendications 11 à 14, comprenant en outre :
arrêter la pompe à air ;
débrancher le connecteur de source lumineuse vis-à-vis de la pompe à air ; et
recouvrir l'endoscope (2) pour un transport,
dans lequel le bouchon d'étanchéité (108), l'ensemble de distribution de détergent (104) et l'appareil de nettoyage (106) restent reliés à l'endoscope (2) lorsqu'il est recouvert.
